# EUROPEAN PATENT APPLICATION

(11) **EP 4 570 238 A1**
(43) Date of publication of application: **18.06.2025**
(21) Application number: 24218921.5
(22) Date of filing: 11.12.2024
(51) Int. Cl.: A61K 9/00, A61K 31/4015, A61K 47/02, A61K 47/10, A61K 47/12, A61K 47/26

(54) **A SACHET FORMULATION COMPRISING BRIVARACETAM**

(30) Priority: 13.12.2023 TR 202317170
(71) Applicant: Sanovel Ilac Sanayi Ve Ticaret Anonim Sirketi, Istanbul (TR)
(72) Inventor: SUNEL, Fatih, Istanbul (TR); BILGEHAN ATAK, Fadime, Istanbul (TR); ATAMAN, Seval, Istanbul (TR); YAZICI, Ozlem, Istanbul (TR)
(74) Representative: Genç Ilhan, Oznur

(57) **Abstract**

The present invention relates to a sachet formulation comprising brivaracetam or a pharmaceutically acceptable salt thereof, and at least one sweetener, wherein the amount of brivaracetam is between 1.0% and 15.0% by weight in the total formulation. The present invention also relates to a simple, rapid, cost effective, time-saving and industrially convenient process of preparing the sachet formulation.

## Description

### Field of the Invention

The present invention relates to a sachet formulation comprising brivaracetam or a pharmaceutically acceptable salt thereof, and at least one sweetener, wherein the amount of brivaracetam is between 1.0% and 15.0% by weight in the total formulation. The present invention also relates to a simple, rapid, cost effective, time-saving and industrially convenient process of preparing the sachet formulation.

### Background of the Invention

The chemical name of brivaracetam is (2S)-2-[(4R)-2-oxo-4-propyl-pyrrolidin-1-yl] butanamide and its chemical structure is shown in the Formula I.

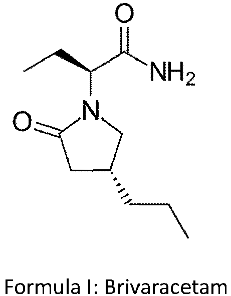

Brivaracetam is a white to off-white crystalline powder. It is very soluble in water, buffer (pH 1.2, 4.5 and 7.4), ethanol, methanol, and glacial acetic acid. It is freely soluble in acetonitrile and acetone and soluble in toluene. It is very slightly soluble in n-hexane. Additionally, brivaracetam is an extremely viscous compound (adhesive capacity).

Brivaracetam is commercially approved in the form of tablets, oral solution and injection, in the USA under the brand name Briviact and are marketed by UCB Pharma.

Due to Brivaracetam's high adhesion properties, we wanted to create a simple and homogeneous sachet formulation by using a several excipients. In this way, the disadvantages of the active substance will be eliminated and patient compliance will be ensured by providing ease of use. Creating sachet formulations provides a more convenient and easier method for active substances that have such disadvantages.

Brivaracetam has low melting point that result in some challenges in tablet coating such as pitting of the coating layer. Since the use of sachet formulation does not contain a coating, it has become advantageous in terms of physical stability as well as patient compliance.

Brivaracetam has a bitter taste so this is a problem that should be overcome. Therefore, it is needed to develop a sachet formulation which has a desired acceptable taste for patients.

A sachet formulation was developed to cater for patients who have difficulty swallowing tablets. The formulation was developed in order to create a solution of acceptable taste, consistency and stability.

### Detailed Description of the Invention

The main object of the present invention provides a sachet formulation comprising brivaracetam having the desired stability, content uniformity and dissolution profile.

Another object of the present invention is to eliminate the problems related to active ingredients and to obtain a solution with pleasant taste when the sachet formulation dissolves in water.

Another object of the present invention is to provide a process of preparing a sachet formulation comprising brivaracetam which is simple and cost-effective process.

The sachet formulations with a good solubility result in a homogenous mixture. Taste is one of the most important parameters governing patient compliance for sachet formulations. It has been surprisingly found that using a sachet formulation comprising brivaracetam with an excipient that is a sweetener, we have achieved a formulation that provides the desired stability, dissolution profile and gives the tablet a good taste.

Also, concerning oral administration, sachet formulations have become an issue with increasing importance in terms of patient compliance as compared to conventional solid dosage forms such as capsules and tablets. This issue is more important in terms of patients having difficulty in swallowing. For these reasons, sachet formulations are one of the advantageous routes for administering the drugs comprising brivaracetam and provide a higher patient compliance along with recommended pharmaceutical therapies.

The term "sachet" will refer to an envelope or bag for a granulate, while "granulate" refers to particles, granulate or spheronised particles. The sachets require no special shaping or molding operations. Thus, the compaction, compression or tamping steps used with other dosage forms are not needed.

According to one embodiment of the invention, a sachet formulation comprises brivaracetam or a pharmaceutically acceptable salt thereof, and at least one pharmaceutically acceptable sweetener, wherein the amount of brivaracetam is between 0.1% and 15.0% by weight in the total formulation.

Preferably, the amount of brivaracetam is between 1.0% and 7.0% by weight in the total formulation. Although, the high adhesion properties of brivaracetam, its use at this ratio provided the desired dissolution profile.

Suitable sweeteners are selected from the group comprising sucralose, sucrose, mannitol, saccharin, neotame, xylose, maltitol, aspartame, maltol, sorbitol, glucose, menthol, peppermint and xylitol or mixtures thereof.

According to an embodiment of the present invention, the sweetener is sucralose or sucrose or mannitol or saccharin or neotame or mixtures thereof. The sweeteners used also help to provide the stability of the sachet form.

According to an embodiment of the present invention, a sachet formulation further comprises at least one pharmaceutically acceptable excipient which is selected from the group comprising flavouring agent, pH agent, acid source, gas generating agent, binders or mixtures thereof.

Suitable flavouring agents are selected from the group comprising orange, lemon, cherry, menthol, peppermint, cinnamon, chocolate, vanillin, strawberry, grape, black currant, raspberry, banana, red fruits, wild berries or mixtures thereof.

According to an embodiment of the present invention, the flavouring agent is orange or lemon or mixtures thereof.

Suitable pH agents are selected from the group comprising monosodium citrate, anhydrous disodium hydrogen phosphate, potassium carbonate, anhydrous sodium dihydrogen phosphate, dibasic potassium sulfate, nicotinic acid, dilute hydrochloric acid, glacial acetic acid, monobasic potassium phosphate, phosphoric acid, adipic acid, sodium acetate, sodium dihydrogen phosphate dihydrate, tribasic sodium phosphate or mixtures thereof.

According to an embodiment of the present invention, the pH agent is monosodium citrate.

Suitable acid sources are selected from the group comprising citric acid anhydrous, malic acid, maleic acid, tartaric acid, nicotinic acid, dilute hydrochloric acid, glacial acetic acid, ascorbic acid, acetylsalicylic acid, lactic acid, adipic acid, tartaric acid or mixtures thereof.

According to this embodiment of the present invention, the acid source is citric acid anhydrous or malic acid or maleic acid or tartaric acid or mixtures thereof.

Suitable gas generating agents are selected from the group comprising sodium bicarbonate, sodium carbonate, calcium carbonate, aluminum potassium sulfate, anhydrous disodium hydrogen phosphate, potassium bicarbonate, anhydrous sodium dihydrogen phosphate, dibasic potassium sulfate, monobasic potassium phosphate, sodium carbonate, sodium glycine carbonate sodium citrate, sodium dihydrogen phosphate dihydrate, tribasic sodium phosphate or mixtures thereof.

According to an embodiment of the present invention, the gas generating agent is sodium bicarbonate or sodium carbonate or calcium carbonate or mixtures thereof.

Suitable binders are selected from the group comprising polyvinylpyrrolidone, hydroxypropyl cellulose, sodium carboxymethyl cellulose, sodium carboxymethyl cellulose, polyethylene glycol, starch, pregelatinized starch, sodium alginate, hydroxypropyl methyl cellulose, carboxy methyl cellulose, methyl cellulose, guar gum, polymethacrylates, methacrylate polymers, polysaccharides, poloxamer, polyoxyethylene-alkyl ether, polydextrose, polyethylene oxide or mixtures thereof.

According to an embodiment of the present invention, the binder is polyvinylpyrrolidone.

Sachet formulation may be achieved using the dry and wet granulation method, resulting in a simple and low-cost production method. We have found that Brivaracetam is extremely prone to sticking and clumping due to the high adhesion during the preparation process, which poses major problems for the appearance and ingredient integrity of the final product. However, thanks to our formulation, we have created a sachet formulation suitable for both wet granulation and dry granulation production. When produced with these processes, there was no problem of content uniformity.

### Example 1;

| **Material** | **% by weight of the total formulation** | **% by weight of the total formulation** |
|---|---|---|
| Brivaracetam | 6.67 | 1-15 |
| Orange | 3.33 | 0.5-10 |
| Saccharin | 23.33 | 15-30 |
| Sucrose | 33.33 | 25-45 |
| Mannitol | 33.33 | 25-45 |
| **TOTAL** | 100 | 100 |

### Method of Manufacture

1) Brivaracetam, orange, saccharin and sucrose, mannitol are mixed
2) Powder is filled into sachets.

### Example 2;

| **Material** | **% by weight of the total formulation** | **% by weight of the total formulation** |
|---|---|---|
| Brivaracetam | 4.00 | 1-15 |
| Sucralose | 1.60 | 0.5-10 |
| Anhydrous citric acid | 10.00 | 5-20 |
| Mannitol | 80.00 | 70-90 |
| Neotame | 0.40 | 0.01-5 |
| Monosodium citrate | 4.00 | 0.5-10 |
| **TOTAL** | 100 | 100 |

### Method of Manufacture

1) Brivaracetam, sucralose, anhydrous citric acid, mannitol, neotame, monosodium sitrate is mixed
2) Powder is filled into sachets

### Example 3;

| **Material** | **% by weight of the total formulation** | **% by weight of the total formulation** |
|---|---|---|
| Brivaracetam | 1.82 | 1-15 |
| Lemon | 12.73 | 5-20 |
| Malic acid | 21.82 | 10-30 |
| Sodium bicarbonate | 9.09 | 1-15 |
| Sucrose | 54.55 | 45-65 |
| **TOTAL** | 100 | 100 |

### Method of Manufacture

1) Brivaracetam, lemon, malic acid, sodium bicarbonate and sucrose are mixed
2) Mixture is filled into sachets.

### Example 4;

| **Material** | **% by weight of the total formulation** | **% by weight of the total formulation** |
|---|---|---|
| Brivaracetam | 4 | 1-15 |
| Mannitol | 60 | 50-70 |
| Maleic acid | 19.2 | 10-30 |
| Calcium Carbonate | 11.2 | 5-20 |
| Lemon flavor | 1.6 | 0.01-5 |
| Sucrose | 4 | 0.5-10 |
| **TOTAL** | 100 | 100 |

### Method of Manufacture

1) Brivaracetam, mannitol, maleic acid, calcium carbonate, lemon flavor and sucrose, are mixed
2) Mixture is filled into sachets.

### Example 5;

| **Material** | **% by weight of the total formulation** | **% by weight of the total formulation** |
|---|---|---|
| Brivaracetam | 4.3 | 1-15 |
| Anhydrous citric acid | 13 | 5-20 |
| Tartaric acid | 8.7 | 1-15 |
| Sodium bicarbonate | 8.7 | 1-15 |
| Sodium carbonate | 17.4 | 5-25 |
| Lemon flavor | 2.2 | 0.5-10 |
| Sucrose | 43.5 | 35-55 |
| Povidone k30 | 2.2 | 0.5-10 |
| **TOTAL** | 100 | 100 |

### Method of Manufacture

1) Brivaracetam, anhydrous citric acid, tartaric acid, lemon, sucrose is mixed
2) Powder is granulated with povidone k30 in water solution and dried
3) Dried granules are sieved and sodium bicarbonate and sodium carbonate are added and mixed
4) Granules are filled into sachets

## Claims

1. A sachet formulation comprises brivaracetam or a pharmaceutically acceptable salt thereof, and at least one pharmaceutically acceptable sweetener, wherein the amount of brivaracetam is between 0.1% and 15.0% by weight in the total formulation.

2. The sachet formulation according to claim 1, wherein sweeteners are selected from the group comprising sucralose, sucrose, mannitol, saccharin, neotame, xylose, maltitol, aspartame, maltol, sorbitol, glucose, menthol, peppermint and xylitol or mixtures thereof.

3. The sachet formulation according to claim 2, wherein the sweetener is sucralose or sucrose or mannitol or saccharin or neotame or mixtures thereof.

4. The sachet formulation according to claim 1, wherein a sachet formulation further comprises at least one pharmaceutically acceptable excipient which is selected from the group comprising flavouring agent, pH agent, acid source, gas generating agent, binders or mixtures thereof.

5. The sachet formulation according to claim 4, wherein flavouring agents are selected from the group comprising orange, lemon, cherry, menthol, peppermint, cinnamon, chocolate, vanillin, strawberry, grape, black currant, raspberry, banana, red fruits, wild berries or mixtures thereof.

6. The sachet formulation according to claim 5, wherein the flavouring agent is orange or lemon.

7. The sachet formulation according to claim 4, wherein pH agents are selected from the group comprising monosodium citrate, anhydrous disodium hydrogen phosphate, potassium carbonate, anhydrous sodium dihydrogen phosphate, dibasic potassium sulfate, nicotinic acid, dilute hydrochloric acid, glacial acetic acid, monobasic potassium phosphate, phosphoric acid, adipic acid, sodium acetate, sodium dihydrogen phosphate dihydrate, tribasic sodium phosphate or mixtures thereof.

8. The sachet formulation according to claim 7, wherein the pH agent is monosodium citrate.

9. The sachet formulation according to claim 4, wherein acid sources are selected from the group comprising citric acid anhydrous, malic acid, maleic acid, tartaric acid, nicotinic acid, dilute hydrochloric acid, glacial acetic acid, ascorbic acid, acetylsalicylic acid, lactic acid, adipic acid, tartaric acid or mixtures thereof.

10. The sachet formulation according to claim 9, wherein the acid source is citric acid anhydrous or malic acid or maleic acid or tartaric acid or mixtures thereof.

11. The sachet formulation according to claim 4, wherein gas generating agents are selected from the group comprising sodium bicarbonate, sodium carbonate, calcium carbonate, aluminum potassium sulfate, anhydrous disodium hydrogen phosphate, potassium bicarbonate, anhydrous sodium dihydrogen phosphate, dibasic potassium sulfate, monobasic potassium phosphate, sodium carbonate, sodium glycine carbonate sodium citrate, sodium dihydrogen phosphate dihydrate, tribasic sodium phosphate or mixtures thereof.

12. The sachet formulation according to claim 11, wherein the gas generating agent is sodium bicarbonate or sodium carbonate or calcium carbonate or mixtures thereof.

13. The sachet formulation according to claim 4, wherein binders are selected from the group comprising polyvinylpyrrolidone, hydroxypropyl cellulose, sodium carboxymethyl cellulose, sodium carboxymethyl cellulose, polyethylene glycol, starch, pregelatinized starch, sodium alginate, hydroxypropyl methyl cellulose, carboxy methyl cellulose, methyl cellulose, guar gum, polymethacrylates, methacrylate polymers, polysaccharides, poloxamer, polyoxyethylene-alkyl ether, polydextrose, polyethylene oxide or mixtures thereof.

14. The sachet formulation according to claim 13, wherein the binder is polyvinylpyrrolidone.
